# EUROPÄISCHE PATENTSCHRIFT

(11) **EP 4 451 078 B1**
(45) Veröffentlichungstag und Bekanntmachung des Hinweises auf die Patenterteilung: **01.07.2026**
(21) Anmeldenummer: 23168257.6
(22) Anmeldetag: 17.04.2023
(51) Int. Cl.: G05B 23/02, G16H 40/40, G06Q 10/20

(54) **FEEDBACK-BASIERTE VORHERSAGE VON MEDIZINGERÄTDEFEKTEN**
FEEDBACK-BASED PREDICTION OF MEDICAL DEVICE DEFECTS
PRÉDICTION DE DÉFAILLANCE DE DISPOSITIF MÉDICAL BASÉE SUR UNE RÉTROACTION

(43) Veröffentlichungstag der Anmeldung: 23.10.2024
(73) Patentinhaber: Fresenius Medical Care Deutschland GmbH, 61352 Bad Homburg v.d.H (DE); Fresenius Medical Care AG, 61352 Bad Homburg (DE)
(72) Erfinder: MÜLLER, Jannik, 61352 Bad Homburg (DE); FÖRSTER, Norman, 61352 Bad Homburg (DE); LUNGHAMER, Bernd, 61352 Bad Homburg (DE); POPOVIC, Radomir, 61352 Bad Homburg (DE)
(74) Vertreter: Bardehle Pagenberg Partnerschaft mbB Patentanwälte Rechtsanwälte

(56) Entgegenhaltungen:
- US-A1- 2016 153 806
- US-A1- 2019 122 761
- US-A1- 2020 310 900
- US-A1- 2022 199 241
- US-A1- 2022 245 799

## Beschreibung

### Gebiet der Erfindung

Die vorliegende Offenbarung betrifft ein computer-implementiertes Verfahren zur Feedbackerzeugung für ein Modell zur Vorhersage von Medizingerätedefekten. Teil der vorliegenden Offenbarung sind zudem ein entsprechendes Modell zur Vorhersage von Medizingerätedefekten, ein Verfahren zum Trainieren dieses Modells sowie eine entsprechende Datenverarbeitungsvorrichtung und ein Computerprogramm.

### Hintergrund

Heutzutage verfügen Medizingeräte über eine Vielzahl an Sensoren, welche eine kontinuierliche Überwachung von Betriebsparametern ermöglichen. Ein besonders kritischer Aspekt liegt hierbei in der Sicherstellung funktionsfähiger und sicherer Medizingeräte im Klinikalltag. Aufgrund von Gerätefehlern, hervorgerufen z.B. durch Komponentenverschleiß, müssen im Rahmen eines guten Risikomanagements üblicherweise mehrere funktionsfähige Medizingeräte vorgehalten werden, sodass im Fehlerfall dennoch die Versorgung von Patienten gewährleistet wird.

Einerseits können so weniger Patienten gleichzeitig versorgt werden, als es mit gegebener Gerätezahl möglich wäre. Andererseits ist ein Zurückstellen zusätzlicher Geräte, z.B. zwei Geräte, unter ungünstigen Umständen unzureichend: In seltenen Fällen treten beispielsweise unerwartet Fehler in mehr als zwei Geräten an einem Tag auf, sodass notwenige Behandlungen verschoben werden müssen.

Ist der Fehlerfall eingetreten, können sich Fehlerzustände als durchaus komplex darstellen. In der Regel reicht dann eine einfache visuelle Überprüfung oder Analyse der Betriebsparameter nicht aus, um eine fundierte Kenntnis der Fehlerursache zu erlangen. Die Ursachenfindung obliegt dann dem beauftragten Servicetechniker, der hierfür einen entsprechenden Erfahrungsschatz und Fachkenntnis mitbringen muss.

Es bedarf somit eines effizienten Verfahrens zur Vorhersage von Medizingerätedefekten, um die Verfügbarkeit und Sicherheit von Medizingeräten sicherzustellen und somit das Eintreten von tatsächlichen Fehlerfällen durch eine rechtzeitige Wartung oder Reparatur zu verringern.

### Zusammenfassung

Aspekte der vorliegenden Offenbarung sind in den beigefügten unabhängigen und abhängigen Ansprüchen dargelegt.

Ein erster Aspekt betrifft ein computer-implementiertes Verfahre zur Vorhersage von Medizingerätedefekten gemäß Patentanspruch 1.

Durch das Übertragen des vorhergesagten Fehlerzustandes an einen Empfänger basierend auf der Komplexität des vorhergesagten Fehlerzustandes wird ein effizienteres Trainieren des Modells erzielt. Dies liegt daran, dass der entsprechende Empfänger in der Lage ist, die Vorhersage des Modells korrekt zu bewerten, wodurch eine belastbare Evidenz zum Anpassen der Parameter des Modells geschaffen wird.

Gemäß einem weiteren Aspekt umfasst das Anpassen des Satzes von Parametern: Bestimmen einer Differenz zwischen dem tatsächlichen Betriebszustand und dem vorhergesagten Betriebszustand, wobei das Anpassen des Satzes von Parametern ferner auf der bestimmten Differenz basiert.

Durch das Bestimmen der Differenz kann ermittelt werden, in welchen Punkten die Vorhersage nicht dem tatsächlichen Betriebszustand entspricht. Somit kann eine zielgerichtete Anpassung der Parameter erfolgen.

Gemäß einem weiteren Aspekt basiert das Bestimmen der Differenz zwischen dem tatsächlichen Betriebszustand und dem vorhergesagten Betriebszustand auf einer erstellten Feedbackdatei, wobei eine Struktur der erstellten Feedbackdatei zumindest teilweise auf der Komplexität des vorhergesagten Fehlerzustandes und/oder auf dem Fähigkeitsprofil des Empfängers basiert, und wobei die Struktur der Feedbackdatei einen Detailgrad der angepassten Feedbackdatei vorgibt.

Die Feedbackdatei ermöglicht einen Feedbackloop, der nicht nur die Komplexität des Fehlerzustandes, sondern auch die Fähigkeiten des Empfängers berücksichtigt. Abhängig von diesen Faktoren kann ein Detailgrad der Feedbackdatei gewählt werden, welcher die bestmögliche Bestimmung der Differenz ermöglicht.

Gemäß einem weiteren Aspekt umfasst die Struktur der Feedbackdatei eine oder mehrere binäre Fragen betreffend den vorhergesagten Fehlerzustand des Medizingerätes einen oder mehrere vordefinierte Zustandsbeschreibungen des Medizingerätes und/oder eine oder mehrere mit dem vorhergesagten Fehlerzustand assoziierten Handlungsanweisungen.

Durch eine Kombination von binären Fragen, vordefinierten Zustandsbeschreibungen und assoziierten Handlungsanweisungen kann ein detaillierter Abgleich zwischen vorhergesagtem Fehlerzustand und tatsächlichem Betriebszustand erfolgen.

Gemäß einem weiteren Aspekt umfasst der zukünftige Fehlerzustand des Medizingerätes: eine Vorhersage einer Ausfallwahrscheinlichkeit des Medizingeräts innerhalb einer vordefinierten zukünftigen Zeitspanne, eine oder mehrere Vorhersagen einer oder mehrerer Ausfallwahrscheinlichkeiten einer oder mehrerer Einzelkomponenten des Medizingerätes und/oder eine oder mehrere Vorhersagen einer oder mehrerer Ursachenwahrscheinlichkeiten für Ausfälle der einen oder mehreren Einzelkomponenten.

Die Abstufungen zwischen den einzelnen Wahrscheinlichkeiten ermöglicht eine detaillierte Ursachenbekämpfung bzw. Bewertung.

Gemäß einem weiteren Aspekt umfasst das Vorhersagen des zukünftigen Fehlerzustandes des Medizingerätes: Treffen, durch ein erstes Teilmodell des Modells, der Vorhersage der Ausfallwahrscheinlichkeit des Medizingeräts innerhalb der vordefinierten zukünftigen Zeitspanne, Treffen, durch eine erste Mehrzahl an Teilmodellen des Modells, der einen oder mehreren Vorhersagen der einen oder mehreren Ausfallwahrscheinlichkeiten der einen oder mehreren Einzelkomponenten des Medizingerätes und/oder Treffen, durch eine zweite Mehrzahl an Teilmodellen des Modells, der einen oder mehreren Vorhersagen der einen oder mehreren Ursachenwahrscheinlichkeiten für die Ausfälle der einen oder mehreren Einzelkomponenten.

Das Zusammensetzen des Modells mittels mehrere Teilmodelle ermöglicht eine Harmonisierung der Vorhersage der einzelnen Wahrscheinlichkeiten.

Gemäß einem weiteren Aspekt umfasst das Anpassen des Satzes von Parametern des Modells: Bestimmen, gemäß des tatsächlichen Betriebszustandes des ersten Teilmodell des Modells, eines Teilmodells der ersten Mehrzahl an Teilmodellen des Modells und/oder eines Teilmodells der zweiten Mehrzahl an Teilmodellen des Modells; und Anpassen eines Satzes von Parametern des bestimmten mindestens einen Teilmodells.

Durch das gezielte Bestimmen des Teilmodells, welches für eine falsche Vorhersage verantwortlich ist, kann ein überflüssiges Trainieren der anderen Teilmodelle verhindert werden. Stattdessen ist es ausreichend, nur den Satz von Parametern des bestimmten Teilmodells anzupassen.

Gemäß einem weiteren Aspekt umfasst das Verfahren ferner: Vortrainieren des Modells mittels einer Mehrzahl an Trainingsdaten als Eingabe für das Modell, wobei eine Trainingsdatei der Mehrzahl an Trainingsdaten umfasst: einen zu einem vergangenen Zeitpunkt eingetretenen Fehlerzustand des Medizingeräts, ein Fehlerprotokoll über einen Betriebszustand des Medizingeräts vor dem vergangenen Zeitpunkt und ein Reparaturprotokoll einschließend Handlungen zur Beseitigung des eingetretenen Fehlerzustandes.

Um eine übermäßige Belastung des Systems zu vermeiden, kann das Modell bereits auf einem entsprechenden Trainingsdatensatz vortrainiert werden. Somit ist das Modell bereits in der Lage, viele Betriebszustände richtig einzuschätzen. Eines Anpassens des Satzes von Parametern bedarf es daher nur noch in speziellen Fällen (z.B. solche Fälle, welche bis dato noch nicht von den Trainingsdaten umfasst sind).

Gemäß einem weiteren Aspekt umfasst das Übertragen: Bestimmen eines Fähigkeitsprofils des Empfängers und Abgleichen der bestimmten Komplexität mit dem Fähigkeitsprofil des Empfängers, wobei das Fähigkeitsprofil zumindest umfasst: Eine Fähigkeit des Empfängers zur Bewertung eines Fehlerzustandes bis zu einer vordefinierten Komplexität.

Durch ein Abgleichen der Komplexität mit dem Fähigkeitsprofil des Empfängers wird vermieden, dass der zu bewertende Fehlerzustand fälschlicherweise an einen Empfänger übertragen wird, welcher nicht die notwendigen Fähigkeiten hat.

Gemäß einem weiteren Aspekt umfasst der aktuelle Betriebszustand des Medizingerätes: Nutzungsdaten des Medizingeräts, technische Gerätedaten des Medizingeräts und/oder Umweltdaten des Medizingeräts, wobei der zukünftige Fehlerzustand des Medizingerätes umfasst: eine Fehlerdiagnose, eine Fehlerwahrscheinlichkeit und/oder eine Zeitspanne.

Ein zweiter Aspekt betrifft ein Modell zur Vorhersage von Medizingerätedefekten gemäß Patentanspruch 10.

Gemäß einem weiteren Aspekt umfasst das Modell: ein erstes Teilmodell des Modells, zur Vorhersage einer Ausfallwahrscheinlichkeit des Medizingeräts innerhalb einer vordefinierten zukünftigen Zeitspanne; eine erste Mehrzahl an Teilmodellen des Modells, zur Vorhersage einer oder mehrerer Ausfallwahrscheinlichkeiten einer oder mehrerer Einzelkomponenten des Medizingerätes; und/oder eine zweite Mehrzahl an Teilmodellen des Modells, zur Vorhersage einer oder mehrerer Ursachenwahrscheinlichkeiten der einen oder mehreren Einzelkomponenten.

Ein dritter Aspekt betrifft eine Datenverarbeitungsvorrichtung umfassend Mittel zum Ausführen des Verfahrens gemäß einem der hierin offenbarten Aspekte und/oder Mittel zum Speichern und Verwenden des Modells gemäß einem der hierin offenbarten Aspekte.

Ein vierter Aspekt betrifft ein Computerprogramm umfassend Anweisungen, welche wenn sie von einem Computer ausgeführt werden, den Computer zum Ausführen des Verfahrens gemäß einem der oben offenbarten Aspekte veranlasst.

US 2016/153806 A1 beschreibt ein System zur Bestimmung eines Gesundheitswerts ("Health Score") für technische Anlagen, das Ausfallwahrscheinlichkeiten berechnet und bei Unterschreiten von Schwellenwerten Warnmeldungen oder Reparaturempfehlungen ausgibt.

US 2022/0245799 A1 fokussiert sich auf die Fehlerbehebung bei Medizingeräten (speziell Dialysemaschinen) mittels computergestützter Modelle, die basierend auf Bilddaten oder historischen Ereignissen Diagnosen erstellen und konkrete Lösungsschritte vorschlagen.

### Kurzbeschreibung der Zeichnungen

Verschiedene Aspekte der vorliegenden Erfindung werden im Folgenden unter Bezugnahme auf die beigefügten Zeichnungen näher beschrieben, ohne dass die vorliegende Erfindung auf die Ausführungsformen dieser Zeichnungen beschränkt ist.
- Fig. 1: zeigt eine Übersicht eines Systems zur Vorhersage von Medizingerätedefekten gemäß Aspekten der vorliegenden Offenbarung.
- Fig. 2: zeigt eine Vorhersage eines Medizingerätedefektes einschließlich Empfängerfeedback gemäß Aspekten der vorliegenden Offenbarung.
- Fig. 3: zeigt einen Ausschnitt einer beispielhafte Trainingsdatei zum Trainieren eines Modells zur Vorhersage von Medizingerätedefekten gemäß Aspekten der vorliegenden Offenbarung.

### Detaillierte Beschreibung bevorzugter Ausführungsformen

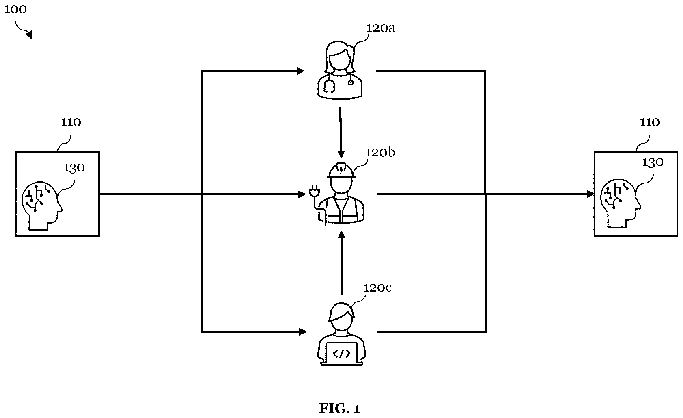

Figur 1 zeigt eine Übersicht eines Systems 100 zur Vorhersage von Medizingerätedefekten gemäß Aspekten der vorliegenden Offenbarung. Innerhalb dieses Systems 100 kann auch ein Verfahren zum Trainieren eines Modells zur Vorhersage von Medizingerätedefekten gemäß Aspekten der vorliegenden Offenbarung erfolgen. Das System 100 kann ein Medizingerät 110 (z.B. ein Dialysegerät etc.) umfassen. Das Medizingerät 110 kann eine Vielzahl von Sensoren zum Überwachen eines Betriebszustandes des Medizingerätes 110 umfassen. Das Medizingerät 110 kann ferner entsprechende Mittel (z.B. Prozessor, Speicher, Sendeempfänger für entsprechende Kommunikationstechnologien wie z.B. MQTTS) zum Sammeln, Verarbeiten, Versenden und Empfangen von Daten (wie z.B. den Sensordaten) umfassen. Das System kann ferner ein Modell 130 zur Vorhersage von Medizingerätedefekten wie z.B. des Medizingeräts 110 umfassen. Das Modell 130 kann sowohl Teil des Medizingerätes 110 sein als auch Teil eines Servers (z.B. Cloudserver). Das Medizingerät kann z.B. eine Datenverarbeitungsvorrichtung umfassend Mittel zum Speichern und Verwenden des Modell 130 beinhalten. In diesem Fall werden die von den Sensoren des Medizingerät 110 gesammelten Sensordaten direkt an die Datenverarbeitungsvorrichtung weitergeleitet, um eine entsprechende Vorhersage zu treffen. Es ist allerdings auch möglich, dass das Modell 130 auf einer Datenverarbeitungsvorrichtung wie z.B. einem Server gehostet wird. In diesem Fall werden die Sensordaten des Medizingerätes 110 zunächst an die Datenverarbeitungsvorrichtung mittels entsprechender Kommunikationstechnologie (z.B. MQTTS, SFTP, HTTPS etc.) übertragen, um anschließend eine entsprechende Vorhersage zu treffen. Die Darstellung in der Fig. 1 dient daher nur der Verdeutlichung. In jedem Fall kann basierend auf einem aktuellen Betriebszustand des Medizingerätes (110) eine Vorhersage durch das Modell (130) über einen zukünftigen Fehlerzustand des Medizingerätes (110) getroffen werden. Zusätzlich kann die Vorhersage auch auf einem oder mehreren historischen Betriebszuständen basieren.

Das System 110 kann zudem einen oder mehrere Empfänger 120a-c umfassen. Der vorhergesagte (zukünftige) Fehlerzustand kann an einen oder mehrere der Empfänger 120a-120c übertragen werden. Ein Empfänger kann anschließend Informationen über einen tatsächlichen Betriebszustand des Medizingerätes 110 erfassen (z.B. mittels einer Bewertung des vorhergesagten Fehlerzustandes basierend auf dem aktuellen Betriebszustand des Medizingerätes 110). Sollte der vorhergesagte Fehlerzustand dem tatsächlichen Betriebszustand des Medizingerätes 110 entsprechen, so kann dies von dem entsprechenden Empfänger bestätigt werden. In diesem Fall wäre ein Anpassen eines Satzes von Parametern des Modells 130 (d.h. ein erneutes Trainieren des Modells 130) nicht notwendig. Sollte jedoch der vorhergesagte Fehlerzustand dem tatsächlichen Betriebszustand des Medizingerätes 110 nicht entsprechen (d.h. es existiert eine Differenz zwischen dem tatsächliche Betriebszustand wie mittels der Informationen über den tatsächlichen Betriebszustand des Medizingerätes angezeigt und dem vorhergesagten Fehlerzustand des Medizingerätes 110), so kann ein Anpassen des Satzes von Parametern des Modells 130 basierend zumindest teilweise auf den Informationen über den tatsächlichen Betriebszustand und dem vorhergesagten Fehlerzustand erfolgen. Die Kommunikation zwischen den einzelnen Entitäten des Systems 100 kann mittels entsprechender Schnittstellenkommunikation erfolgen. Hierzu kann beispielsweise eine REST-API als Schnittstelle implementiert werden mittels welcher z.B. der vorhergesagte Fehlerzustand an einen oder mehrere der Empfänger 120a-c übertragen werden kann. Andererseits kann mittels eines entsprechenden Schnittstellenaufrufs wiederum die Information über den tatsächlichen Betriebszustand von dem entsprechenden Empfänger 120a-c an das Modell 130 übertragen werden, wodurch ein etwaiges Anpassen des Satzes von Parametern des Modells ermöglicht wird.

Bei den Empfängern 120a-c kann es sich z.B. um eine Pflegefachkraft 120a, einen Servicetechniker 120b und Fernwartungspersonal 120c handeln. Dabei kann jeder Empfänger 120a-c mit einem entsprechenden Fähigkeitsprofil assoziiert sein. Dieses kann z.B. eine Fähigkeit des Empfängers zur Bewertung eines Fehlerzustandes bis zu einer vordefinierten Komplexität umfassen. Entsprechend kann das Übertragen des vorhergesagten (zukünftigen) Fehlerzustandes des Medizingerätes (110) an einen oder mehrere Empfänger 120a-c von einer Komplexität des vorhergesagten Fehlerzustandes abhängen. Ein Fähigkeitsprofil der Pflegefachkraft 120a kann z.B. eine Fähigkeit zur Bewertung eines Fehlerzustandes von niedriger Komplexität umfassen. Ein Fähigkeitsprofil des Fernwartungspersonals 120c kann z.B. eine Fähigkeit zur Bewertung eines Fehlerzustandes von mittlerer Komplexität umfassen. Ein Fähigkeitsprofil des Servicetechnikers 120b kann z.B. eine Fähigkeit zur Bewertung eines Fehlerzustandes von hoher Komplexität umfassen.

Ein Fehlerzustand kann eine niedrige Komplexität aufweisen, wenn der Fehlerzustand z.B. visuell überprüfbar ist (z.B. ob ein Bibag-Konnektor korrekt angebracht ist oder einen Riss hat). Dieser Fehlerzustand könnte dann an jeden der Empfänger 120a-c übertragen werden, denn jeder Empfänger hat gemäß seines Fähigkeitsprofils die Fähigkeit zur Bewertung eines Fehlerzustandes von (mindestens) niedriger Komplexität.

Ein Fehlerzustand kann eine mittlere Komplexität aufweisen, wenn der Fehlerzustand z.B. nicht visuell überprüfbar ist (z.B. wenn es eines Auslesens eines Gerätefehlerspeichers des Medizingerätes 110 bedarf). Dieser Fehlerzustand könnte dann nur an die Empfänger 120b-c übertragen werden, denn diese haben gemäß ihrer Fähigkeitsprofile die Fähigkeit zur Bewertung eines Fehlerzustandes von mittlerer Komplexität, während der Empfänger 120a nur Fehlerzustände von niedriger Komplexität bewerten kann und somit nicht in Betracht kommt.

Ein Fehlerzustand kann eine hohe Komplexität aufweisen, wenn der Fehlerzustand z.B. ein Auseinanderbauen des Medizingerätes erfordert. Dieser Fehlerzustand könnte dann nur an den Empfänger 120b übertragen werden, denn nur dieser hat gemäß seines Fähigkeitsprofils die Fähigkeit zur Bewertung eines Fehlerzustandes von hoher Komplexität, während die Empfänger 120a-b nur Fehlerzustände von niedriger bzw. mittlerer Komplexität bewerten können. Im Falle, dass ein Fehlerzustand von zu hoher Komplexität für den entsprechenden Empfänger trotzdem an diesen übertragen wurde, kann dieser an den passenden Empfänger (d.h. den Empfänger, welcher die Komplexität bewerten kann) weitergeleitet werden. Dies kann z.B. aufgrund einer Fehleinschätzung des Modells 130 erfolgt sein (z.B. wenn das Modell 130 noch nicht ausreichend trainiert wurde). Das Bestimmen der Komplexität eines Fehlerzustandes kann z.B. mittels einer Look-Up Tabelle erfolgen, welche für jeden Fehlerzustand eine entsprechende Komplexität beinhaltet. Das Bestimmen der Komplexität kann aber auch auf Basis einer zusätzlichen Ausgabe des Modells 130 erfolgen.

Ob eine Differenz zwischen dem tatsächlichen Betriebszustand und dem vorhergesagten Fehlerzustand existiert, kann auf einer erstellten Feedbackdatei basieren. Die Feedbackdatei kann dabei eine Struktur aufweisen, welche auf der Komplexität des vorhergesagten Fehlerzustandes und/oder auf dem Fähigkeitsprofil des Empfängers 120a-c basiert. Die Struktur der Feedbackdatei kann hierbei einen Detailgrad der Feedbackdatei vorgeben. So kann eine Feedbackdatei für einen Fehlerzustand von hoher Komplexität eine Struktur von höherem Detailgrad aufweisen als eine Feedbackdatei für einen Fehlerzustand von niedriger Komplexität. Ebenfalls kann eine Feedbackdatei, welche z.B. an den Servicetechniker 120b übertragen wird, eine Struktur von höherem Detailgrad aufweisen als eine Feedbackdatei, welche z.B. an die Pflegefachkraft 120a übertragen wird. Dabei kann die Struktur der Feedbackdatei z.B. eine oder mehrere binäre Fragen betreffend den vorhergesagten Fehlerzustand beinhalten (z.B. "Bibag-Konnektor korrekt angebracht? Ja/Nein"). Die Anzahl an binären Fragen kann abhängig von der Komplexität des Fehlerzustandes sein.

Die Struktur der Feedbackdatei kann ebenfalls eine oder mehrere vordefinierte Zustandsbeschreibungen des Medizingerätes beinhalten (z.B. "Zahnradpumpe verschlissen", "Ausfall eines Verdichters"). Durch eine Kombination einer oder mehrerer dieser Zustandsbeschreibungen kann auch ein komplexer Sachverhalt beschrieben werden. Außerdem können einzelne Zustandsbeschreibungen semantisch hervorgehoben werden. Sollte z.B. der Servicetechniker 120b der Auffassung sein, dass hauptsächlich eine verschlissene Zahnradpumpe für einen Defekt des Medizingerätes 110 verantwortlich ist, obwohl das Modell 130 nur den Ausfall des Verdichters im vorhergesagten Fehlerzustand aufgeführt hat, kann er dies mittels entsprechender Syntax (z.B. Reihenfolge, Schriftart etc.) verdeutlichen. So kann z.B. der Hauptgrund als erstes aufgeführt werden und erst anschließend ein vermeintlicher Nebengrund ("Zahnradpumpe verschlissen" und "Ausfall eines Verdichters). Durch diese optimierte Art der Feedbackerzeugung kann die Vorhersagequalität des Modells 130 verbessert werden. Zum Beispiel kann so die Anzahl der "richtig negativen" bzw. "falsch positiven" reduziert werden, wodurch sich die Vorhersagequalität (z.B. in Bezug auf Recall und Precision) des Modells verbessert.

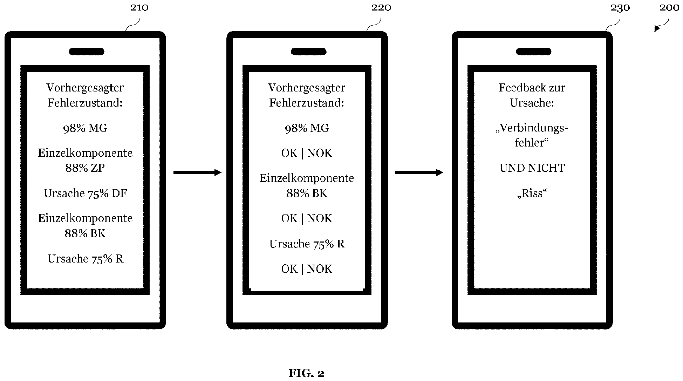

Figur 2 zeigt eine Vorhersage eines Medizingerätedefektes einschließlich Empfängerfeedback gemäß Aspekten der vorliegenden Offenbarung. Als ein Medizingerätedefekt kann jede Ursache beschrieben werden, welche die Behandlung eines Patienten mit dem Medizingerät 110 verhindert oder einschränkt.

Abschnitt 210 der Figur 2 zeigt einen vorhergesagten Fehlerzustand gemäß Aspekten der vorliegenden Offenbarung.

Dieser kann eine Vorhersage einer Ausfallwahrscheinlichkeit des Medizingerätes 110 innerhalb einer vordefinierten zukünftigen Zeitspanne (z.B. Tage, Wochen etc.) umfassen. Hierzu kann das Modell 130 ein erstes Teilmodell umfassen, welches die Vorhersagen zu der Ausfallwahrscheinlichkeit innerhalb der vordefinierten zukünftigen Zeitspanne trifft. In dem in Abschnitt 210 gezeigten Beispiel hat das Modell 130 bzw. das erste Teilmodell eine Ausfallwahrscheinlichkeit des Medizingerätes (MG) 110 von 98% innerhalb der vordefinierten zukünftigen Zeitspanne (z.B. 20 Tage) vorhergesagt. Es kann zudem vordefiniert sein, ab welcher Wahrscheinlichkeit ein vorhergesagter Fehlerzustand an einen Empfänger 120a-c übertragen wird. So kann z.B. festgelegt werden (z.B. von einem Systemingenieur oder einem der Empfänger 120a-c), dass ein Fehlerzustand erst ab einer Wahrscheinlichkeit (d.h. Ausfallwahrscheinlichkeit(en) und/oder Ursachenwahrscheinlichkeit) von 20% oder 50% übertragen wird.

Zusätzlich oder alternativ kann der vorhergesagte Fehlerzustand eine oder mehrere Vorhersagen einer oder mehrerer Ausfallwahrscheinlichkeiten einer oder mehrerer Einzelkomponenten des Medizingerätes 110 umfassen. Hierzu kann das Model 130 eine erste Mehrzahl an Teilmodellen umfassen, welche die Vorhersagen der einen oder mehreren Ausfallwahrscheinlichkeiten der einen oder mehreren Einzelkomponenten des Medizingerätes 100 treffen. Dadurch kann eine Aussage darüber getroffen werden, welche Einzelkomponente des Medizingerätes 110 repariert bzw. ausgetauscht werden soll. Die Teilmodelle der ersten Mehrzahl an Teilmodellen können somit als binär Modelle (z.B. Klassifizierer) verstanden werden, wobei ein Teilmodell der ersten Mehrzahl an Teilmodellen für die binäre Vorhersage einer Einzelkomponente verantwortlich ist. In dem in Abschnitt 210 gezeigten Beispiel hat ein erstes Teilmodell der ersten Mehrzahl an Teilmodellen des Modells 130 eine Ausfallwahrscheinlichkeit von 88% der Zahnradpumpe (ZP) vorhergesagt. Ein zweites Teilmodel der ersten Mehrzahl an Teilmodellen des Modells 130 hat zudem eine Ausfallwahrscheinlichkeit von 88% des Bibag-Konnektors (BK) vorhergesagt.

Zusätzlich oder alternativ kann der vorhergesagte Fehlerzustand eine oder mehrere Vorhersagen einer oder mehrerer Ursachenwahrscheinlichkeiten für Ausfälle der einen oder mehreren Einzelkomponenten umfassen. Hierzu kann das Model 130 eine zweite Mehrzahl an Teilmodellen umfassen, welche die Vorhersagen der einen oder mehreren Ursachenwahrscheinlichkeiten für die Ausfälle der einen oder mehreren Einzelkomponenten des Medizingerätes 110 treffen. Die Teilmodelle der zweiten Mehrzahl an Teilmodellen können somit als binär Modelle (z.B. Klassifizierer) verstanden werden, wobei ein Teilmodell der zweiten Mehrzahl an Teilmodellen für die binäre Vorhersage einer Ursachenwahrscheinlichkeit für den Ausfall der entsprechenden Einzelkomponente verantwortlich ist. In dem in Abschnitt 210 gezeigten Beispiel hat ein erstes Teilmodell der zweiten Mehrzahl von Teilmodellen des Modells 130 eine 75% Ursachenwahrscheinlichkeit für den Ausfall der entsprechenden Einzelkomponente (in diesem Beispiel der Zahnradpumpe ZP) aufgrund einer Desinfektionsflüssigkeit (DF) vorhergesagt und ein zweites Teilmodell der zweiten Mehrzahl an Teilmodellen des Modells 130 eine 75% Ursachenwahrscheinlichkeit für den Ausfall der entsprechenden Einzelkomponente (hier des BK) aufgrund eines Risses (R).

Abschnitt 220 zeigt eine beispielhafte Darstellung einer Feedbackdatei des Fehlerzustandes gemäß Aspekten der vorliegenden Offenbarung.

Diese kann beispielsweise auf einem mobilen Endgerät (z.B. Smartphone, Tablet, Laptop, Smartwatch) oder einem stationären Endgerät (z.B. Desktop-PC) oder einem anderen Datenverarbeitungsgerät des Empfängers 120a-c dargestellt werden. Wie hierin beschrieben, kann die Struktur der Feedbackdatei von der Komplexität des Fehlerzustandes und/oder von dem Fähigkeitsprofils des entsprechenden Empfängers 120a-c abhängen. Im gezeigten Beispiel 220 handelts es sich um einen Fehlerzustand, welcher eine 98% Ausfallwahrscheinlichkeit des Medizingerätes (MG) 110 beinhaltet. Zudem beinhaltet der Fehlerzustand eine 88% Ausfallwahrscheinlichkeit einer Einzelkomponente (hier dem Bibag-Konnektor BK) des Medizingerätes 110, welcher Grund für die Ausfallwahrscheinlichkeit von 98% des Medizingerätes 110 sein könnte. Zusätzlich beinhaltet der Fehlerzustand eine Ursachenwahrscheinlichkeit für den Ausfall der Einzelkomponente von 75% (hier z.B. ein Riss (R) in dem Bibag-Konnektor). Die Komplexität dieses Fehlerzustandes kann als niedrig bestimmt werden, da dieser z.B. visuell bewertbar ist. Entsprechend könnte dieser Fehlerzustand an jeden der Empfänger 120a-c übertragen werden. Wie im Beispiel gezeigt, weist die Struktur der Feedbackdatei hierzu zunächst drei binäre Fragen auf, welche entweder mit "OK" (d.h. die Vorhersage ist zutreffend) oder "NOK" (d.h. die Vorhersage trifft nicht zu) geantwortet werden kann. Sollten alle drei Fragen mit "OK" beantwortet werden, so entspricht der vorhergesagte Fehlerzustand dem tatsächlichen Betriebszustand, d.h. es besteht keine Differenz. In diesem Fall wäre ein Anpassen des Satzes von Parametern des Modells 130 nicht notwendig. Sollte jedoch eine der Vorhersagen nicht zutreffen, d.h. es besteht eine Differenz zwischen dem vorhergesagten Fehlerzustand und dem tatsächlichen Betriebszustand, kann diese Information dem Modell 130 mitgeteilt werden und der Satz von Parametern entsprechend angepasst werden.

Entsprechend kann auch das Anpassen des Satzes von Parametern des Modells 130 variieren. Zunächst kann gemäß des tatsächlichen Betriebszustandes bzw. den entsprechenden Informationen bestimmt werden, welche(s) Teilmodell(e) des Modells 130 angepasst werden soll(en). Hierzu kann z.B. basierend auf der Differenz zwischen vorhergesagtem Fehlerzustand und dem tatsächlichen Betriebszustand ermittelt werden, bei welcher Vorhersage (z.B. Vorhersage der Ausfallwahrscheinlichkeit, Vorhersage einer Ausfallwahrscheinlichkeit einer Einzelkomponente oder Vorhersage einer Ursachenwahrscheinlichkeit) das Modell 130 bzw. das entsprechende Teilmodell falsch lag. Anschließenden kann ein Satz von Parametern des bestimmten (Teil-)Modells erfolgen.

Hierzu zeigt Abschnitt 230 der Figur 2 eine entsprechende Eingabemöglichkeit. Sollte eine Vorhersage des Modells 130 bzw. eines Teilmodells nicht korrekt sein, so kann der Empfänger 120a-c dies im gezeigten Beispiel dadurch mitteilen, dass er zunächst die entsprechende binäre Frage mit "NOK" beantwortet. Sollte der Empfänger 120a-c z.B. den Bibag-Konnektor auf einen Riss kontrolliert und dabei festgestellt haben, dass kein Riss erkennbar ist, aber der Bibag-Konnektor nicht richtig eingesteckt ist, so würde der Empfänger 120a-c zunächst die vorhergesagte Ursachenwahrscheinlichkeit für den Ausfall der Einzelkomponente (hier dem Bibag-Konnektor) mit "NOK" beantworten. Anschließend kann er die Informationen über den tatsächlichen Betriebszustand in die Feedbackdatei eintragen. So kann er z.B. mittels vordefinierten Zustandsbeschreibungen den tatsächlichen Betriebszustand des Medizingerätes 110 beschreiben. In dem genannten Beispiel könnte er zunächst die tatsächliche Ursache nennen und anschließend mittels einer entsprechenden logischen Verbindung die vorhergesagte Ursache verneinen (z.B. "Verbindungsfehler" UND NICHT "Riss"). Empfängt das Modell 130 diese Informationen, kann anschließend ein Satz von Parametern des Modells 130 basierend auf dem vorhergesagten Fehlerzustand und den empfangen Informationen über den tatsächlichen Betriebszustand des Medizingerätes 110 angepasst werden. In dem Beispiel könnte z.B. bestimmt werden, dass es eine Differenz zwischen dem vorhergesagten Fehlerzustand und dem tatsächlichen Betriebszustand gibt, nämlich, dass die Ursache für den Ausfall des Bibag-Konnektors ein Verbindungsfehler und nicht wie vorhergesagt ein Riss ist. Basierend auf dieser Information kann dann zum einen das Teilmodell bestimmt werden, welches für die Ursachenvorhersage "Verbindungsfehler" zuständig ist, und zum anderen das Teilmodell bestimmt werden, welches für die Ursachenvorhersage "Riss" zuständig ist. Anschließend kann ein Satz von Parametern der bestimmten Teilmodelle angepasst werden (d.h. neu trainiert werden).

Somit kann das Modell 130 als eine Zusammensetzung (z.B. Ensemble) von mehreren Teilmodellen verstanden werden, wobei jedes Teilmodell des Modells 130 für eine bestimmte Vorhersage verantwortlich (d.h. trainiert) ist. So kann z.B. ein erstes Teilmodell der ersten Mehrzahl an Teilmodellen ausschließlich für die Vorhersage der Ausfallwahrscheinlichkeit einer bestimmten Einzelkomponente (z.B. Zahnradpumpe) des Medizingerätes 110 konfiguriert sein und ein zweites Teilmodell der ersten Mehrzahl an Teilmodellen ausschließlich für die Vorhersage der Ausfallwahrscheinlichkeit einer anderen Einzelkomponente (z.B. Bibag-Konnektor). Wiederum kann ein erstes Teilmodell der zweiten Mehrzahl an Teilmodellen ausschließlich für die Vorhersage der Ursachenwahrscheinlichkeit (z.B. Desinfektionsflüssigkeit) für den Ausfall einer bestimmten Einzelkomponente (z.B. Zahnradpumpe) konfiguriert sein, ein zweites Teilmodell der zweiten Mehrzahl an Teilmodellen für die Vorhersage einer anderen Ursachenwahrscheinlichkeit (z.B. Zahnradverschliss) der Zahnradpumpe und ein drittes Teilmodell der zweiten Mehrzahl an Teilmodellen wiederum für die Vorhersage einer bestimmten Ursachenwahrscheinlichkeit (z.B. Riss) für den Ausfall einer anderen Einzelkomponente (z.B. Bibag-Konnektor).

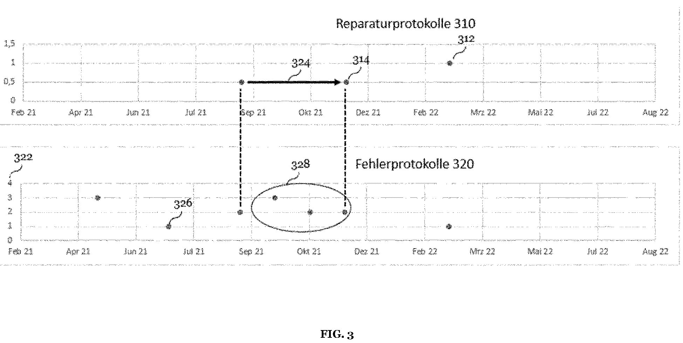

Figur 3 zeigt einen Ausschnitt 300 von beispielhaften Trainingsdaten zum Trainieren eines Modells 130 zur Vorhersage von Medizingerätedefekten gemäß Aspekten der vorliegenden Offenbarung. Hierbei kann das Trainieren auch ein Vortrainieren des Modells 130 basierend auf den Trainingsdaten umfassen.

Dazu wird eine Mehrzahl an Trainingsdaten als Eingabe für das Modell 130 verwendet. Die Mehrzahl an Trainingsdaten kann gemäß eines Training-Test-Verhältnisses (z.B. 80% zu 20%) aufgeteilt werden. Eine Trainingsdatei der Mehrzahl an Trainingsdaten kann dabei einen zu einem vergangenen Zeitpunkt eingetretenen Fehlerzustand des Medizingerätes, ein Fehlerprotokoll 320 über einen Betriebszustand des Medizingerätes vor dem vergangenen Zeitpunkt und ein Reparaturprotokoll 312 einschließend Handlungen zur Beseitigung des eingetretenen Fehlerzustandes aufweisen. Bei dem Modell 130 bzw. den entsprechenden Teilmodellen kann es sich um einen XGBoost-Klassifizierer mit z.B. 100 Schätzern und einer maximalen Tiefe von 4 handeln. Teil des Trainings kann eine Validierung mittels "K-Fold Cross Validation" umfassen.

Im oberen Teil der Figur 3 ist eine beispielhafte Darstellung von Reparaturprotokollen 310 dargestellt. Dabei steht ein Punkt für jeweils ein erstelltes Reparaturprotokoll 312. Das hervorgehobene Reparaturprotokoll 312 enthält beispielsweise einen Zeitpunkt der durchgeführten Reparatur (in diesem Beispiel den 15. Februar 2022) sowie eine oder mehrere durchgeführte Handlungen zur Beseitigung des eingetretenen Fehlerzustandes des Medizingerätes 110. In diesem Beispiel beinhaltet das Reparaturprotokoll 312 beispielsweise eine durchgeführte Handlung, wobei eine durchgeführte Handlung jeweils einen entsprechenden Handlungscode, eine betroffene Einzelkomponente des Medizingerätes 110 sowie eine kurze Beschreibung der Handlung beinhaltet. In dem gezeigten Beispiel beinhaltet das Reparaturprotokoll 312 eine erste durchgeführte Handlung, welche den Handlungscode "1", die betroffenen Einzelkomponente "Bigbag-Konnektor" und die Beschreibung "Austausch" beinhaltet.

In dem unteren Teil der Figur 3 ist eine beispielhafte Darstellung von Fehlerprotokollen 320 gezeigt. Wie für die in dem oberen Teil der Figur 3 gezeigten Reparaturprotokolle 310, spiegelt die X-Achse einen Zeitstrahl (hier beispielhaft von Februar 2021 bis August 2022). Im Fall der Fehlerprotokolle 320 zeigt die Y-Achse eine Mehrzahl von Fehlercodes 322 (z.B. Fehlercode 1, Fehlercode 2 usw. bis Fehlercode 4). Dabei steht jeder Fehlercode der Mehrzahl von Fehlercodes 322 für eine entsprechende Fehlerart bzw. Ursache. Darüber hinaus kann jedes Auftreten eines Fehlers bzw. Fehlercodes 326 mit zusätzlichen Informationen verbunden sein. Diese Informationen können eine Zeitspanne (z.B. eine Anzahl an Tagen), in welcher das Medizingerät 110 im Einsatz war, bevor der Fehler aufgetreten ist, eine Seriennummer des Medizingerätes 110, sowie einen Zeitpunkt des Auftretens beinhalten. Zusätzlich kann der aufgetretenen Fehler gelabelt sein (z.B. binär), wobei das Label anzeigt, ob aufgrund des Fehlers ein Ausfall des Medizingerätes 110 und/oder ein Ausfall einer Einzelkomponente des Medizingerätes 110 erfolgt ist. Im Falle eines binären Labels steht eine 1 für einen Ausfall und eine 0 für keinen Ausfall. Die Labels können entweder manuell erstellt sein oder approximiert. Z.B. können alle Fehler, welche innerhalb einer vordefinierten Zeit, z.B. 20 Tage, vor einem Ausfall aufgetreten sind mit 1 gelabelt werden, und Fehler, welche nicht innerhalb der vordefinierten Zeit aufgetreten sind mit 0.

Diese über die Zeit gesammelten Fehlerprotokolle können als Zeitreihe interpretiert werden und mittels der zugehörigen Reparaturprotokolle 310 in Teilzeitreihen 324 aufgeteilt werden. Somit besteht eine Teilzeitreihe 324 aus einer Teilmenge 328 der Fehlerprotokolle 320 und endet mit einem entsprechenden Reparaturprotokoll 314. Jede dieser Teilzeitreihen kann mit einer entsprechenden ID versehen werden. Von jeder Teilzeitreihe 324 können (zählerbasierte) Merkmale extrahiert werden, wie z.B. die Anzahl an Vorkommnissen eines entsprechenden Fehlercodes 322 innerhalb der Teilzeitreihe 324. Sind mehrere Reparaturprotokolle 310 innerhalb kurzer Zeit erfolgt, so können die zugehörigen Teilzeitreihen zu einer zusammengefügten Teilzeitreihe verbunden werden, da die einzelnen Teilzeitreihen sonst zum Teil zu kurz und somit zu wenig verwertbare Fehlerprotokolle 320 beinhalten würden. Somit entsteht eine längere Teilzeitreihe, welche mehr verwertbare Fehlerprotokolle 320 beinhaltet. Als das zugehörige Reparaturprotokoll 310 wird dann das zuletzt erfolgte Reparaturprotokoll 310 der mehreren Reparaturprotokolle gewählt. Es ist aber auch möglich, die Reparaturprotokolle zu einem Reparaturprotokoll zu vereinen.

Ein Betriebszustand (d.h. der aktuelle Betriebszustand sowie der Betriebszustand vor dem vergangenen Zeitpunkt) kann Nutzungsdaten des Medizingerätes 110, technische Gerätedaten des Medizingerätes 110 und/oder Umweltdaten (z.B. Temperatur des Umfelds) des Medizingerätes 130 umfassen. Diese Daten können in einer entsprechenden Datenbank, welche z.B. Teil des Systems 100 ist, gespeichert sein und bei Bedarf (z.B. für ein (Vor-)Training) abgerufen werden.

Nutzungsdaten des Medizingerätes 110 können beispielweise den Gerätenamen, die Geräteposition (z.B. Behandlungsort oder allgemeine Koordinaten wie GPS), den Gerätehersteller, die Seriennummer, die Gerätesoftware, die IP-Adresse des Medizingerätes 110 innerhalb des entsprechenden Netzwerks bzw. Systems 100, eine Gerätemodulkonfiguration (z.B. Informationen über zusätzliche Module, welche an das Medizingerät 110 angebaut wurden), einen Behandlungsmodus des Medizingerätes (z.B. Informationen über eine Vielzahl von möglichen Behandlungsmodi, welche das Medizingerät 110 ausführen kann, Information über den zuletzt verwendeten Behandlungsmodus etc.), Angaben über die letzte Behandlung (z.B. Start und Endzeitpunkt der Behandlung mittels des Medizingerätes, effektive Behandlungsdauer wie z.B. die effektive Dialysedauer), Sitzungszeit (z.B. Start- und Endzeitpunkt), Informationen über Warnungen während der Sitzung (z.B. eine Liste von Warnungen und/oder Störungen), und/oder Informationen über eine Behandlungsphase umfassen.

Technische Gerätedaten des Medizingerätes 110 können beispielsweise einen Satz an Parametern von Dienstverbindungsmerkmalen wie z.B. Betriebsdauer (z.B. in Stunden oder Minuten) des Medizingerätes 110 und/oder entsprechender Module und/oder Einzelkomponenten, Hydraulikinformationen (z.B. gemessener Unterdruck Pa bei einer kalibrierten Drehzahl gemittelt über eine Dauer von z.B. 5 Sekunden, gemessener Unterdruck Pa bei maximaler Drehzahl von z.B: 3000 rpm gemittelt über eine Dauer von z.B. 5 Sekunden und/oder eine Drehzahl rpm bei einem vorbestimmten Unterdruck z.B. von 0,81-0,85 bar, wobei die Drehzahl z.B. von einem Entgasungspumpenantrieb gemessen wird) und/oder eine Anzahl an Hüben einer Dosierpumpe (z.B. gemessen mittels eines Hardwarezählers) umfassen. Zudem können die technischen Gerätedaten die in einem Fehlerspeicher des Medizingerätes gesammelten Informationen (z.B. das Fehlerprotokoll über einen Betriebszustand des Medizingerätes 110 vor dem vergangenen Zeitpunkt), Sensorparameter des Medizingerätes 110 sowie anderweitig erfasste Parameter (z.B. Informationen, welche automatisch bei einer Desinfektionsroutine des Medizingerätes 110 erfasst werden) enthalten. Tabelle 1 zeigt eine beispielhafte Übersicht eines Satzes an Parametern von Dienstverbindungsmerkmalen, wobei jeweils der Name des Parameters, eine Beschreibung, die Einheit, ein Multiplikator sowie ein Systemkontext (z.B. DB-Variable, Systemparameter etc.) enthalten ist.

Reparaturprotokolle können beispielsweise Informationen wie Zeit und Datum der Reparatur, Ort der Reparatur (z.B. Klinik), und/oder Informationen über die reparierten Einzelkomponenten enthalten. Die Handlungen zur Beseitigung des eingetretenen Fehlerzustandes können eine Beschreibung der Handlung beinhalten. Somit kann erkannt werden, ob typidentische Einzelkomponenten in unterschiedlichen Modulen des Medizingerätes verwendet werden bzw. verwendet werden können (z.B. kann eine Pumpe sowohl für das Getriebe, die Entlüftung als auch die Strömungspumpe des Medizingerätes verwendet werden). Die Handlung zur Beseitigung kann zudem mit einem Handlungscode assoziiert sein, wodurch Handlungen identifiziert werden können.

Das/die Verfahren gemäß der vorliegenden Erfindung kann/können in Form eines Computerprogramms implementiert werden, das auf jeder geeigneten Datenverarbeitungsvorrichtung ausgeführt werden kann, die entsprechend konfigurierte Mittel (z. B. einen Speicher und einen oder mehrere mit dem Speicher operativ gekoppelte Prozessoren) umfasst. Das Computerprogramm kann als computerausführbare Befehle auf einem nichttransitorischen computerlesbaren Medium gespeichert sein.

Ausführungsformen der vorliegenden Offenbarung können in verschiedenen Formen realisiert werden. Zum Beispiel kann die vorliegende Erfindung in einigen Ausführungsformen als computerimplementiertes Verfahren, als computerlesbares Speichermedium oder als Computersystem realisiert werden.

In einigen Ausführungsformen kann ein nichttransitorisches, computerlesbares Speichermedium so konfiguriert sein, dass es Programmanweisungen und/oder Daten speichert, wobei die Programmanweisungen, wenn sie von einem Computersystem ausgeführt werden, das Computersystem veranlassen, ein Verfahren durchzuführen, z. B. eine der hier beschriebenen Verfahrensausführungen oder eine beliebige Kombination der hier beschriebenen Verfahrensausführungen oder eine beliebige Teilmenge einer der hier beschriebenen Verfahrensausführungen oder eine beliebige Kombination solcher Teilmengen.

In einigen Ausführungsformen kann eine Rechenvorrichtung so konfiguriert sein, dass sie einen Prozessor (oder einen Satz von Prozessoren) und ein Speichermedium enthält, wobei das Speichermedium Programmanweisungen speichert, wobei der Prozessor so konfiguriert ist, dass er die Programmanweisungen aus dem Speichermedium liest und ausführt, wobei die Programmanweisungen ausführbar sind, um eine der verschiedenen hier beschriebenen Verfahrensausführungen (oder eine beliebige Kombination der hier beschriebenen Verfahrensausführungen oder eine beliebige Teilmenge einer der hier beschriebenen Verfahrensausführungen oder eine beliebige Kombination solcher Teilmengen) zu implementieren. Die Vorrichtung kann in verschiedensten Formen realisiert werden.

## Patentansprüche

1. Computer-implementiertes Verfahren zur Vorhersage von Medizingerätedefekten, umfassend:
Vorhersagen, durch ein bereits trainiertes Modell, eines zukünftigen Fehlerzustandes eines Medizingerätes basierend auf einem aktuellen Betriebszustand des Medizingerätes;
wobei das Modell durch ein Verfahren zum Trainieren eines Modells zur Vorhersage von Medizingerätedefekten trainiert wurde, welches folgende Schritte umfasst:
Vorhersagen, durch das Modell (130), eines zukünftigen Fehlerzustandes eines Medizingerätes (110) basierend auf einem aktuellen Betriebszustand des Medizingerätes (110);
Bestimmen einer Komplexität des vorhergesagten Fehlerzustandes;
Übertragen des vorhergesagten Fehlerzustandes an einen Empfänger (120a; 120b; 120c) basierend zumindest teilweise auf der Komplexität des vorhergesagten Fehlerzustandes, wobei das Übertragen umfasst:
Bestimmen des Fähigkeitsprofils des Empfängers; und
Abgleichen der bestimmten Komplexität mit dem Fähigkeitsprofil des Empfängers;
wobei das Fähigkeitsprofil zumindest umfasst:
eine Fähigkeit des Empfängers zur Bewertung eines Fehlerzustandes bis zu einer vordefinierten Komplexität;
Empfangen von Informationen über einen tatsächlichen Betriebszustand des Medizingerätes (110); und
Anpassen eines Satzes von Parametern des Modells (130) basierend zumindest teilweise auf den Informationen über den tatsächlichen Betriebszustand und dem vorhergesagten Fehlerzustand des Medizingerätes (110).

2. Verfahren nach dem vorhergehenden Anspruch, wobei das Anpassen des Satzes von Parametern des Modells umfasst:
Bestimmen einer Differenz zwischen dem tatsächlichen Betriebszustand und dem vorhergesagten Fehlerzustand;
wobei das Anpassen des Satzes von Parametern ferner auf der bestimmten Differenz basiert.

3. Verfahren nach dem vorhergehenden Anspruch, wobei das Bestimmen der Differenz zwischen dem tatsächlichen Betriebszustand und dem vorhergesagten Fehlerzustand auf einer erstellten Feedbackdatei basiert;
wobei eine Struktur der erstellten Feedbackdatei zumindest teilweise auf der Komplexität des vorhergesagten Fehlerzustandes und/oder auf einem Fähigkeitsprofil des Empfängers basiert; und
wobei die Struktur der Feedbackdatei einen Detailgrad der Feedbackdatei vorgibt.

4. Verfahren nach dem vorhergehenden Anspruch, wobei die Struktur der Feedbackdatei umfasst:
eine oder mehrere binäre Fragen betreffend den vorhergesagten Fehlerzustand des Medizingerätes; und/oder
einen oder mehrere vordefinierte Zustandsbeschreibungen des Medizingerätes; und/oder
eine oder mehrere mit dem vorhergesagten Fehlerzustand assoziierten Handlungsanweisungen.

5. Verfahren nach einem der vorhergehenden Ansprüche, wobei der zukünftige Fehlerzustand des Medizingerätes umfasst:
eine Vorhersage einer Ausfallwahrscheinlichkeit des Medizingeräts innerhalb einer vordefinierten zukünftigen Zeitspanne; und/oder
eine oder mehrere Vorhersagen einer oder mehrerer Ausfallwahrscheinlichkeiten einer oder mehrerer Einzelkomponenten des Medizingerätes; und/oder
eine oder mehrere Vorhersagen einer oder mehrerer Ursachenwahrscheinlichkeiten für Ausfälle der einen oder mehreren Einzelkomponenten.

6. Verfahren nach dem vorhergehenden Anspruch, wobei das Vorhersagen des zukünftigen Fehlerzustandes des Medizingerätes umfasst:
Treffen, durch ein erstes Teilmodell des Modells, der Vorhersage der Ausfallwahrscheinlichkeit des Medizingeräts innerhalb der vordefinierten zukünftigen Zeitspanne; und/oder
Treffen, durch eine erste Mehrzahl an Teilmodellen des Modells, der einen oder mehreren Vorhersagen der einen oder mehreren Ausfallwahrscheinlichkeiten der einen oder mehreren Einzelkomponenten des Medizingerätes; und/oder
Treffen, durch eine zweite Mehrzahl an Teilmodellen des Modells, der einen oder mehreren Vorhersagen der einen oder mehreren Ursachenwahrscheinlichkeiten für die Ausfälle der einen oder mehreren Einzelkomponenten.

7. Verfahren nach dem vorhergehenden Anspruch, wobei das Anpassen des Satzes von Parametern des Modells umfasst:
Bestimmen gemäß des tatsächlichen Betriebszustandes des ersten Teilmodells des Modells, eines Teilmodells der ersten Mehrzahl an Teilmodellen des Modells und/oder eines Teilmodells der zweiten Mehrzahl an Teilmodellen des Modells; und
Anpassen eines Satzes von Parametern des bestimmten mindestens einen Teilmodells.

8. Verfahren nach einem der vorhergehenden Ansprüche, wobei das Verfahren zum Trainieren ferner umfasst:
Vortrainieren des Modells mittels einer Mehrzahl an Trainingsdaten als Eingabe für das Modell;
wobei eine Trainingsdatei der Mehrzahl an Trainingsdaten umfasst:
einen zu einem vergangenen Zeitpunkt eingetretenen Fehlerzustand des Medizingeräts;
ein Fehlerprotokoll über einen Betriebszustand des Medizingeräts vor dem vergangenen Zeitpunkt; und
ein Reparaturprotokoll einschließend Handlungen zur Beseitigung des eingetretenen Fehlerzustandes.

9. Verfahren nach einem der Ansprüche 1- 8, wobei der aktuelle Betriebszustand des Medizingerätes umfasst:
Nutzungsdaten des Medizingeräts, technische Gerätedaten des Medizingeräts und/oder Umweltdaten des Medizingeräts; und/oder
wobei der zukünftige Fehlerzustand des Medizingerätes umfasst:
eine Fehlerdiagnose, eine Fehlerwahrscheinlichkeit und/oder eine Zeitspanne.

10. Modell zur Vorhersage von Medizingerätedefekten, wobei das Modell gemäß dem Verfahren zum Trainieren eines Modells zur Vorhersage von Medizingerätedefekten nach einem der Ansprüche 1-9 trainiert wurde.

11. Modell nach Anspruch 10 umfassend:
ein erstes Teilmodell des Modells, zur Vorhersage einer Ausfallwahrscheinlichkeit des Medizingeräts innerhalb einer vordefinierten zukünftigen Zeitspanne; und/oder
eine erste Mehrzahl an Teilmodellen des Modells, zur Vorhersage einer oder mehrerer Ausfallwahrscheinlichkeiten einer oder mehrerer Einzelkomponenten des Medizingerätes; und/oder
eine zweite Mehrzahl an Teilmodellen des Modells, zur Vorhersage einer oder mehrerer Ursachenwahrscheinlichkeiten der einen oder mehreren Einzelkomponenten.

12. Datenverarbeitungsvorrichtung umfassend Mittel zum Ausführen des Verfahrens gemäß einem der Ansprüche 1-9; und/oder Mittel zum Speichern und Verwenden des Modells gemäß einem der Ansprüche 10-11.

13. Computerprogramm umfassend Anweisungen, welche, wenn sie von einem Computer ausgeführt werden den Computer zum Ausführen des Verfahrens gemäß einem der Ansprüche 1-9 veranlasst.

## Claims

1. A computer-implemented method for predicting medical device defects, comprising:
predicting, by an already trained model, a future failure state of a medical device based on a current operating state of the medical device;
wherein the model has been trained by a method for training a model for predicting medical device defects, comprising the steps of:
predicting, by the model (130), a future failure state of a medical device (110) based on a current operating state of the medical device (110);
determining a complexity of the predicted failure state;
transmitting the predicted failure state to a receiver (120a; 120b; 120c) based at least in part on the complexity of the predicted failure state, wherein the transmitting comprises:
determining the capability profile of the receiver; and
matching the determined complexity with the capability profile of the receiver;
wherein the capability profile at least comprises:
a capability of the receiver to evaluate a failure state up to a predefined complexity;
receiving information about an actual operating state of the medical device (110); and
adjusting a set of parameters of the model (130) based at least in part on the information about the actual operating state and the predicted failure state of the medical device (110).

2. The method according to the preceding claim, wherein adjusting the set of parameters of the model comprises:
determining a difference between the actual operating state and the predicted failure state;
wherein adjusting the set of parameters is further based on the determined difference.

3. The method according to the preceding claim, wherein determining the difference between the actual operating state and the predicted failure state is based on a created feedback file;
wherein a structure of the created feedback file is based at least in part on the complexity of the predicted failure state and/or on a capability profile of the receiver; and
wherein the structure of the feedback file specifies a level of detail of the feedback file.

4. The method according to the preceding claim, wherein the structure of the feedback file comprises:
one or more binary questions regarding the predicted failure state of the medical device; and/or
one or more predefined state descriptions of the medical device; and/or
one or more action instructions associated with the predicted failure state.

5. The method according to any one of the preceding claims, wherein the future failure state of the medical device comprises:
a prediction of a failure probability of the medical device within a predefined future time period; and/or
one or more predictions of one or more failure probabilities of one or more individual components of the medical device; and/or
one or more predictions of one or more cause probabilities for failures of the one or more individual components.

6. The method according to the preceding claim, wherein predicting the future failure state of the medical device comprises:
making, by a first partial model of the model, the prediction of the failure probability of the medical device within the predefined future time period; and/or
making, by a first plurality of partial models of the model, the one or more predictions of the one or more failure probabilities of the one or more individual components of the medical device; and/or
making, by a second plurality of partial models of the model, the one or more predictions of the one or more cause probabilities for the failures of the one or more individual components.

7. The method according to the preceding claim, wherein adjusting the set of parameters of the model comprises:
determining, according to the actual operating state of the first partial model of the model, a partial model of the first plurality of partial models of the model and/or a partial model of the second plurality of partial models of the model; and
adjusting a set of parameters of the determined at least one partial model.

8. The method according to any one of the preceding claims, wherein the method for training further comprises:
pre-training the model by means of a plurality of training data as input for the model;
wherein a training file of the plurality of training data comprises:
a failure state of the medical device that occurred at a past time;
a failure log about an operating state of the medical device prior to the past time; and
a repair log including actions to eliminate the occurred failure state.

9. The method according to any one of claims 1-8, wherein the current operating state of the medical device comprises:
usage data of the medical device, technical device data of the medical device, and/or environmental data of the medical device; and/or
wherein the future failure state of the medical device comprises:
a failure diagnosis, a failure probability, and/or a time period.

10. A model for predicting medical device defects, wherein the model has been trained according to the method for training a model for predicting medical device defects according to any one of claims 1-9.

11. The model according to claim 10, comprising:
a first partial model of the model, for predicting a failure probability of the medical device within a predefined future time period; and/or
a first plurality of partial models of the model, for predicting one or more failure probabilities of one or more individual components of the medical device; and/or
a second plurality of partial models of the model, for predicting one or more cause probabilities of the one or more individual components.

12. A data processing device comprising means for performing the method according to any one of claims 1-9; and/or means for storing and using the model according to any one of claims 10-11.

13. A computer program comprising instructions which, when executed by a computer, cause the computer to perform the method according to any one of claims 1-9.

## Revendications

1. Procédé mis en œuvre par ordinateur destiné à prédire des défaillances de dispositifs médicaux, comprenant :
la prédiction, par un modèle déjà entraîné, d'un état d'erreur futur d'un dispositif médical sur la base d'un état de fonctionnement actuel du dispositif médical ;
dans lequel le modèle a été entraîné par un procédé d'entraînement d'un modèle de prédiction de défaillances de dispositifs médicaux, lequel comprend des étapes suivantes :
la prédiction, par le modèle (130), d'un état d'erreur futur d'un dispositif médical (110) sur la base d'un état de fonctionnement actuel du dispositif médical (110) ;
la détermination d'une complexité de l'état d'erreur prédit ;
la transmission de l'état d'erreur prédit à un destinataire (120a ; 120b ; 120c) sur la base au moins en partie de la complexité de l'état d'erreur prédit, dans lequel la transmission comprend :
la détermination du profil de compétences du destinataire ; et
la comparaison de la complexité déterminée au profil de compétences du destinataire ;
dans lequel le profil de compétences comprend au moins :
une capacité du destinataire à évaluer un état d'erreur jusqu'à une complexité prédéfinie ;
la réception d'informations sur un état de fonctionnement réel du dispositif médical (110) ; et
l'adaptation d'un ensemble de paramètres du modèle (130) sur la base au moins en partie des informations sur l'état de fonctionnement réel et de l'état d'erreur prédit du dispositif médical (110).

2. Procédé selon la revendication précédente, dans lequel l'adaptation de l'ensemble de paramètres du modèle comprend :
la détermination d'une différence entre l'état de fonctionnement réel et l'état d'erreur prédit ;
dans lequel l'adaptation de l'ensemble de paramètres se base en outre sur la différence déterminée.

3. Procédé selon la revendication précédente, dans lequel la détermination de la différence entre l'état de fonctionnement réel et l'état d'erreur prédit se base sur un fichier de retour créé ;
dans lequel une structure du fichier de retour créé se base au moins en partie sur la complexité de l'état d'erreur prédit et/ou sur un profil de compétences du destinataire ; et
dans lequel la structure du fichier de retour spécifie un degré de détail du fichier de retour.

4. Procédé selon la revendication précédente, dans lequel la structure du fichier de retour comprend :
une ou plusieurs questions binaires concernant l'état d'erreur prédit du dispositif médical ; et/ou
une ou plusieurs descriptions d'état prédéfinies du dispositif médical ; et/ou une ou plusieurs instructions de manipulation associées à l'état d'erreur prédit.

5. Procédé selon l'une des revendications précédentes, dans lequel l'état d'erreur futur du dispositif médical comprend :
une prédiction d'une probabilité de panne du dispositif médical dans un laps de temps futur prédéfini ; et/ou
une ou plusieurs prédictions d'une ou plusieurs probabilités de panne d'un ou plusieurs composants individuels du dispositif médical ; et/ou
une ou plusieurs prédictions d'une ou plusieurs
probabilité des causes de pannes des un ou plusieurs composants individuels.

6. Procédé selon la revendication précédente, dans lequel la prédiction de l'état d'erreur futur du dispositif médical comprend :
la réalisation, par un premier sous-modèle du modèle, de la prédiction de la probabilité de panne du dispositif médical dans le laps de temps futur prédéfini ; et/ou
la réalisation, par une première pluralité de sous-modèles du modèle, d'une ou de plusieurs prédictions des une ou plusieurs probabilités de panne des un ou plusieurs composants individuels du dispositif médical ; et/ou
la réalisation, par une deuxième pluralité de sous-modèles du modèle, des une ou plusieurs prédictions des une ou plusieurs probabilités de cause de pannes des un ou plusieurs composants individuels.

7. Procédé selon la revendication précédente, dans lequel l'adaptation de l'ensemble de paramètres du modèle comprend :
la détermination, en fonction de l'état de fonctionnement réel du premier sous-modèle du modèle, d'un sous-modèle de la première pluralité de sous-modèles du modèle et/ou d'un sous-modèle de la deuxième pluralité de sous-modèles du modèle ; et
l'adaptation d'un ensemble de paramètres de l'au moins un sous-modèle déterminé.

8. Procédé selon l'une des revendications précédentes, dans lequel le procédé d'entraînement comprend en outre :
le pré-entraînement du modèle au moyen d'une pluralité de données d'entraînement en tant qu'entrée pour le modèle ;
dans lequel un fichier d'entraînement de la pluralité de données d'entraînement comprend :
un état d'erreur du dispositif médical survenue dans le passé ;
un journal d'erreurs sur un état de fonctionnement du dispositif médical antérieur ; et
un protocole de réparation incluant les mesures à prendre pour éliminer l'état d'erreur survenu.

9. Procédé selon l'une des revendications 1 à 8, dans lequel l'état de fonctionnement actuel du dispositif médical comprend :
des données d'utilisation du dispositif médical, des données techniques du dispositif médical et/ou des données environnementales du dispositif médical ; et/ou dans lequel l'état d'erreur futur du dispositif médical comprend :
un diagnostic d'erreur, une probabilité d'erreur et/ou un laps de temps.

10. Modèle de prédiction de défaillances de dispositifs médicaux, dans lequel le modèle a été entraîné selon le procédé d'entraînement d'un modèle de prédiction de défaillances de dispositifs médicaux selon l'une des revendications 1 à 9.

11. Modèle selon la revendication 10 comprenant :
un premier sous-modèle du modèle pour prédire une probabilité de panne du dispositif médical dans un laps de temps futur prédéfini ; et/ou une première pluralité de sous-modèles du modèle, pour prédire une ou plusieurs probabilités de panne d'un ou de plusieurs composants individuels du dispositif médical ; et/ou
une deuxième pluralité de sous-modèles du modèle, pour prédire une ou plusieurs probabilités de cause des un ou plusieurs composants individuels.

12. Dispositif de traitement de données comprenant des moyens d'exécution du procédé selon l'une des revendications 1 à 9 ; et/ou des moyens de stockage et d'utilisation du modèle selon l'une des revendications 10 à 11.

13. Programme informatique comprenant des instructions qui, lorsqu'elles sont exécutées par un ordinateur, amènent l'ordinateur à exécuter le procédé selon l'une des revendications 1 à 9.
